(19) Europäisches Patentamt
European Patent Office
Office européen des brevets

(11) Publication number: 0 013 817 B1

(12) EUROPEAN PATENT SPECIFICATION

(45) Date of publication of patent specification: 15.06.83

(51) Int. Cl.³: C 07 D 409/12, A 61 K 31/415

(21) Application number: 79302921.6

(22) Date of filing: 17.12.79

(54) **1-Imidazol-2-ylidene-3-thienyl-urea derivatives, processes therefor, and pharmaceutical compositions.**

(30) Priority: 19.01.79 US 4675

(43) Date of publication of application: 06.08.80 Bulletin 80/16

(45) Publication of the grant of the patent: 15.06.83 Bulletin 83/24

(84) Designated Contracting States: AT BE CH DE FR GB IT LU NL SE

(56) References cited: US-A-3 983 135

(73) Proprietor: ICI AMERICAS INC
Concord Pike & New Murphy Road
Wilmington Delaware 19897 (US)

(72) Inventor: Bare, Thomas Michael
1078 Windy Knoll Road
West Chester, PA 19380 (US)

(74) Representative: Allerton, Roy et al,
Imperial Chemical Industries PLC Legal
Department: Patents Thames House North
Millbank London SW1P 4QG (GB)

**The file contains technical information submitted after the application was filed and not included in this specification**

EP 0 013 817 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# 1-imidazol-2-ylidene-3-thienyl-urea derivatives, processes therefor, and pharmaceutical compositions

This invention relates to 1-imidazol-2-ylidene-3- thienyl-urea derivatives, to processes for their manufacture, and to pharmaceutical compositions containing them. The said compounds have useful anxiolytic (anti-anxiety) activity, and some of them act to block acid secretions.

In the Journal of Medicinal Chemistry, 1978, *21*, No. 10, 1045—1054 there are described *inter alia* compounds of the formula (I):

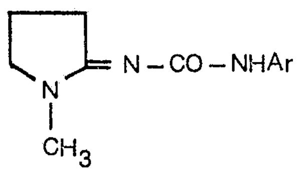

I

wherein Ar stands for a phenyl, substituted phenyl, 3-pyridyl, 2-thienyl or 2-furyl radical. Some of the phenyl and substituted phenyl derivatives are said to have anxiolytic or muscle relaxant properties. However, the compounds wherein Ar stands for a 3-pyridyl, 2-thienyl or 2-furyl radical are said to be of no pharmacological interest.

In United States patent specifications Nos. 3,983,135 and 4,025,517 there are described *inter alia* compounds of the formula (II):

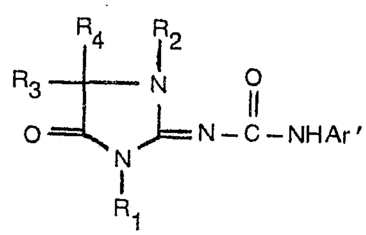

II

wherein $R_1$ stands for hydrogen or a lower alkyl radical, $R_2$ stands for hydrogen or a lower alkyl radical, $R_3$ stands for hydrogen or an aryl radical, $R_4$ stands for hydrogen or an aryl or arylalkyl radical, provided that $R_3$ and $R_4$ are other than 2,6-disubstituted aryl radicals, and Ar′ stands for an aryl radical. The term "aryl" is said to include both unsubstituted and substituted phenyl radicals. The compounds are said to be useful as anti-secretory agents and central nervous system depressants, for example anti-anxiety agents.

We have now discovered that corresponding new compounds in which Ar′ in the formula (II) stands for an unsubstituted or halogen substituted thienyl radical are significantly more active as anxiolytic agents than the abovementioned known compounds of the formula (II).

According to the invention there are provided compounds of the formula (III):

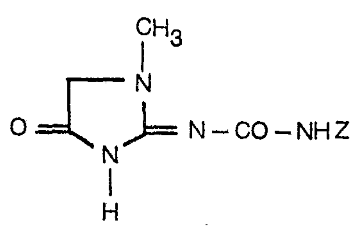

III

wherein Z stands for a thienyl radical or a thienyl radical substituted with a halogen atom, and pharmaceutically-acceptable acid-addition salts thereof.

It is to be understood that the compounds of the formula (III) may exist in another tautomeric form (V):

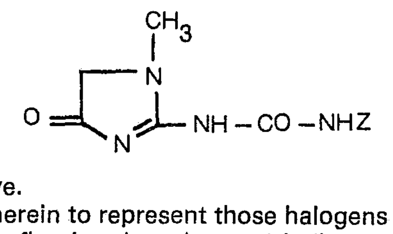

IV

Z has the meaning stated above.

The term halogen is used herein to represent those halogens having an atomic weight of no more than 127 and includes chlorine, fluorine, bromine and iodine.

One embodiment of the invention comprises compounds of the formula (III) wherein Z stands for a thienyl radical which is substituted with a chlorine atom.

Suitable pharmaceutically-acceptable acid-addition salts of the invention include, for example, hydrochlorides, hydrobromides, phosphates, sulphates, citrates, acetates and maleates.

A particularly preferred compound of the invention is 1-(2-chloro-4-thienyl)-3-(tetrahydro-1-methyl-4-oxo-1H-imidazol-2-ylidene)urea.

According to a further feature of the invention there is provided a process for the manufacture of the compounds of the formula (III), wherein Z has the meaning stated above, and pharmaceutically-acceptable acid-addition salts thereof, which comprise reacting a compound of the formula (V):

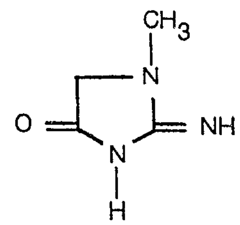

V

with an isocyanate of the formula ZNCO, wherein Z has the meaning states above.

The process may conveniently be carried out by reacting the compound of the formula (V) with an equivalent amount or a slight excess of the isocyanate in an anhydrous aprotic organic solvent, for example dimethylformamide (DMF), dimethylsulphoxide (DMSO), tetrahydrofuran (THF) or toluene, at 25 to 100°C for 1 to 10 hours. The product can be isolated by evaporating the reaction solvents *in vacuo* and purifying the residue by conventional techniques, or by pouring the reaction mixture into water and collecting the precipitated product. This collected product is purified by conventional procedures.

The reactants needed to manufacture the compounds of the invention are either commercially available or they can readily be prepared by well known methods described in the literature. Thus, several heterocyclic isocyanates of the formula ZNCO are known, but those that are not may be prepared by known methods [see Lombardino et al., J. Med. Chem., *7* 97 (1964), or Singleton et al., J. Amer. Chem. Soc., *60*, 540 (1938)].

As indicated above, the compounds of the present invention are considered to be useful in the treatment of anxiety in living animals, particularly mammals.

The general class of compounds known as the benzodiazepines is widely prescribed for the treatment of anxiety. These compounds are excellent anxiolytic agents, but do have associated with them a number of ancillary activities such as sedation, muscle relaxation, anti-convulsant and hypnotic properties. Since the benzodiazepines are central nervous system depressants, they also potentiate the depressant effects of alcohol and other depressants such as the barbiturates. Consequently, patients using the benzodiazepines must be warned against taking excessive doses of these compounds at the same time as alcohol, and of the additive effects of sedative drugs. In the laboratory the Geller-Seifter Conflict test and the Shock-Induced Suppression of Drinking (SSD) test are considered good laboratory models for human anxiety. The sedative and muscle relaxant component of the benzodiazepines can be correlated with a general central nervous system (CNS) battery screen which includes the forced motor activity (FMA) test (to measure neuromuscular impairment) and several anti-convulsant tests (strychnine, metrazol, electroshock) which gave an indication of muscle relaxant activity. Therefore, any anxiolytic agent (as determined by the Geller-Seifter or SSD test) can also be tested for potential sedative and muscle relaxant side-effects by using the CNS battery. Obviously, anxiolytic agents which have good activity in the Geller-Seifter or SSD test and a low degree of sedative and muscle relaxant effects as measured by the CNS battery would be the compounds which are desired. In standard laboratory tests the compounds of the present invention, in general, as described herein demonstrate their ability to fulfill this goal, i.e. anxiolytic activity with little or no sedative action.

Among the tests conducted to demonstrate the anxiolytic activity of the present compounds was the Shock-Induced Suppression of Drinking (Rats) (SDD) Test which was carried out as follows:

Male rats in the weight range of 250 to 280 grams are water-deprived for 48 hours and food-deprived for 24 hours before testing. The rats are orally intubated (5ml/kg) with the test compound (based on mg/kg body weight). The vehicle control group of rats is also intubated by mouth. Also, a dose of 18mg/kg of chlordiazepoxide is orally administered to a positive control of group of rats. Randomization is utilized in dosing. The rats are returned to the cage for one hour. Sixty minutes after drug administration, the rat is quietly removed from its cage and the hind feet wiped with a 10% solution of EEG electrode cream. The rat is placed on the floor in the chamber facing the licking tube. The animal is allowed 5 minutes to make 20 licking responses and receive the first shock (0.5 mA). If this does not occur, the animal is removed and eliminated from the study. If 20 licking responses are made, the animal is permitted an additional 3 minutes during which time each 20th lick is paired with a 0.5 mA shock. This period is automatically started, counted, and terminated. The number of licks and shocks is recorded. The activity of the compound tested is evaluated by comparing the mean shocks of the group dosed with the test compound to both the mean shocks of the vehicle and positive control groups. The higher the number of shocks received the higher the anti-conflict or anti-anxiety activity the compound has.

In general, testing of the compounds of the present invention in rats in the above described SSD test indicates that the effective anxiolytic dosage in living animals, when administered orally, is from about 5mg/kg to 200mg/kg body weight, with a more preferred range being from about 7mg/kg to 100mg/kg body weight.

The compound of Example 1, which is a preferred compound, exhibited about the same level of activity in the above described SSD test when administered at 25mg/kg body weight as chlordiazepoxide when administered at 18mg/kg body weight. The compound of Example 2, another preferred compound, when dosed at 12.5mg/kg body weight in the SSD test exhibited about the same level of activity as chlordiazepoxide administered at 18mg/kg. The compound of Example 6, which is a particularly preferred compound, when dosed at 6.25mg/kg body weight in the SSD test exhibited about the same level of activity as chlordiazepoxide administered at 18mg/kg body weight.

Based on the activities of the compounds of the invention is standard animal tests and a comparison of these with the activities of presently known anxiolytic agents in the same tests, it is concluded that the pharmaceutical compositions of this invention (see below) may, in general, be administered to man for the treatment of anxiety at an oral dose of between about 5mg and 500mg of active ingredient, the composition being administered 1 to 4 times a day. A more preferred oral dosage for man is considered to be from about 5mg to 250mg of active ingredient 1 to 4 times a day. It will, however, be appreciated that the amount of the compound to be administered will vary depending on the degree of anxiety to be dealt with and the compound used.

Some of the subject compounds tested were also found to block acid secretion under the standard laboratory test procedure—the pylorus ligated rat (Shay Rat, WSR). Under the test utilized, five rats each weighing about 170 grams are dosed with each compound to be tested at a dose of 50 milligrams per kilogram body weight. 59.5 milligrams of each compound to be tested is dispersed in 7 millilitres of an aqueous solution containing 0.5% weight/volume of hydroxypropylmethylcellulose and 0.1% weight/ volume of TWEEN 80 polyoxyethylene(20)sorbitan mono-oleate. One millilitre of this resulting dispersion containing a compound to be tested is given intraduodenally to each of five rats to be tested at the time of surgery. The rats are anaesthetized with 37.5 milligrams per kilogram body weight of Brevital (methohexital sodium) given by intraperitoneal injection. An incision about one inch long is made in the centre of the abdomen of the rat going caudal from the base of the sternum. The incision is made with a scalpel in mid-line through to the body cavity. The duodenum is pulled through the open wound until the pyloric area is out. Then a nylon tie is placed under the antral stomach just above the pylorus and pulled tight to create a stricture. The intestines are then replaced in the body cavity and the wound closed with a wound clipper followed by a liberal painting with collodion. After a 4 hour period each rat is sacrificed by cervical dislocation and the stomach is pulled out by the oesophagus gripped by a haemostat. The stomach is then cut away from the intestine and membranes from the other end of the stomach and the stomach is cut open along the greater curvature while holding above a funnel positioned above a graduated centrifuged tube to collect the content of the stomach. The material is centrifuged at top speed for 8 to 12 minutes and an aliquot portion is titrated to determine the acid concentration and total acid output. A control of five rats not dosed with a drug but given the vehicle is run with each group of rats tested. Results are reported in percent inhibition versus the controls.

The compounds of Examples 1 and 3 when tested according to the above described procedure exhibited very significant acid anti-secretory activity. In general, based on test results it is considered that the effective acid blocking dosage of the compounds of this invention when administered orally is from about 10mg/kg to 200mg/kg body weight, preferably from 10mg/kg to 100mg/kg body weight.

According to yet a further feature of the invention there are provided pharmaceutical compositions comprising a compound of the formula (III), Z has the meaning stated above, or a pharmaceutically-acceptable acid-addition salt thereof, together with a pharmaceutically-acceptable diluent or carrier.

The pharmaceutical compositions of the invention contain conventional diluents or carriers and they may be manufactured by conventional procedures. They may be in a form suitable for oral administration, for example tablets, capsules, syrups, elixirs or suspensions, or in a form suitable for parenteral or rectal administration. It is to be understood that the size of, for example, a tablet or capsule of the invention, or the strength of a dosage form of the invention, may be suitably varied in order to satisfy any particular requirements. For example, a dosage unit of the invention may contain from 1 to 50mg of active ingredient together with the pharmaceutically-acceptable diluent or carrier.

The invention is illustrated by the following Examples:—

## Example 1

To 9.4g (83mM) of creatinine in 100ml of anhydrous DMF was added with stirring a solution of 9.2g (75mM) of 2-thienyl isocyanate in 60ml of toluene. After the addition was completed, the mixture was stirred at 55°C for 1 hour, cooled, and the solvents removed in vacuo. The residue was crystallized first from ethanol and then ethyl acetate (treated with charcoal) to give 1-(tetrahydro-1-methyl-4-oxo-1H-imidazol-2-ylidene)-3-(2-thienyl)urea, m.p. 191—192°C (dec.).

Example 2

To a stirred suspension of 2.6g (23mM) of creatinine in 50ml of anhydrous DMF was added a solution of 3.0g (23mM) of 3-thienyl isocyanate in 25ml of toluene. The resulting mixture was heated at 75°C for 4.5 hours, cooled and added to 150ml of water. The resulting precipitate was collected and crystallized from ethyl acetate to give 1-(tetrahydro-1-methyl-4-oxo-1H-imidazol-2-ylidene)-3-(3-thienyl)urea, m.p. 194—195°C.

Example 3

To 3.0g (26.5mM) of creatinine in 50ml of anhydrous DMF was added with stirring a solution of 4-chloro-2-thienyl isocyanate in 25ml of toluene. After stirring at 65°C for 5 hours, the mixture was cooled, added to 200ml of water and filtered to separate a precipitate, which was crystallized from ethanol and then ethyl acetate to give 1-(4-chloro-2-thienyl)-3-(tetrahydro-1-methyl-4-oxo-1H-imidazol-2-ylidene)urea containing 1/8th mole of ethyl acetate of crystallization, m.p. 203—204°C. (dec.).

Example 4

To a stirred suspension of 3.0g (26mM) of creatinine in 30ml of anhydrous DMF was added a solution of 5.0g of 5-bromo-2-thienyl isocyanate in 15ml of toluene. The resulting mixture was heated at 75°C for 3 hours, cooled and poured into 200ml. of water. The precipitate was collected, washed with ethanol and ether, and air-dried. The solid was dissolved in 250ml hot acetone, treated with charcoal, filtered, and the filtrate diluted with water (250ml). The resulting precipitate was collected, washed with acetone and then ether. Air-drying gave 1-(5-bromo-2-thienyl)-3-(tetrahydro-1-methyl-4-oxo-1H-imidazol-2-ylidene)urea, m.p. 192—193°C (dec.).

Example 5

To a stirred suspension of 0.8g (7.1mM) of creatinine in 10ml of anhydrous DMF was added a solution of 1.12g (5.5mM) of 3-bromo-2-thienylisocyanate in 5ml of toluene. The resulting mixture was heated at 65°C for 5 hours, cooled and poured into a mixture of 200ml of water and 25ml of toluene. The precipitate was collected and crystallized from ethyl acetate to give 1-(3-bromo-2-thienyl)-3-(tetrahydro-1-methyl-4-oxo-1H-imidazol-2-ylindene)urea, m.p. 192—193°C (dec.).

Example 6

To a stirred suspension of 2.5g (22.5mM) of creatinine in 30ml of anhydrous DMF was added a solution of 2.87g (18mM) of 2-chloro-4-thienyl isocyanate in 20ml of toluene. The resulting mixture was heated at 80°C for 3.5 hours, cooled and poured into a mixture of 250ml of water and 50ml of toluene. The precipitate was collected and crystallized from ethyl acetate to give 1-(2-chloro-4-thienyl-3-(tetrahydro-1-methyl-4-oxo-1H-imidazol-2-ylidene)urea, m.p. 198—200°C.

Example 7

To a stirred suspension of 2.5g (22.5mM) of creatinine in 30ml of anhydrous DMF was added a solution of 2.87g (18mM) of 5-chloro-2-thienyl isocyanate in 15ml of toluene. The resulting mixture was heated at 80°C for 3.5 hours, cooled and poured into a mixture of 250ml of water and 50ml of toluene. The precipitate was collected and crystallized from ethyl acetate to give 1-(5-chloro-2-thienyl)-3-(tetrahydro-1-methyl-4-oxo-1H-imidazol-2-ylidene)urea, m.p. 204—205°C

**Claims for the Contracting States: BE, CH, DE, FR, GB, IT, LU, NL, SE.**

1. A compound of the formula (III):

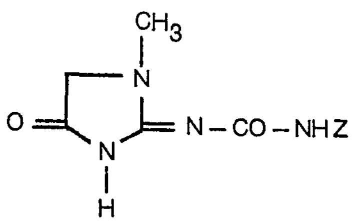

III

characterised in that Z stands for a thienyl radical or a thienyl radical substituted with a halogen atom, or a pharmaceutically-acceptable acid-addition salt thereof.

2. A compound as claimed in claim 1 characterised in that Z stands for a thienyl radical which is substituted with a chlorine atom.

3. A compound as claimed in claim 1 characterised in that it is 1-(2-chloro-4-thienyl)-3-(tetrahydro-1-methyl-4-oxo-1H-imidazol-2-ylidene)urea.

4. A salt as claimed in claim 1 characterised in that it is a hydrochloride, hydrobromide, phosphate, sulphate, citrate, acetate or maleate.

5. A process for the manufacture of a compound claimed in claim 1, characterised in that a compound of the formula (V):

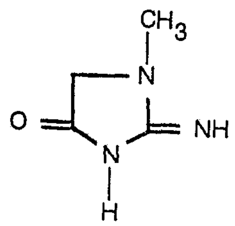

V

is reacted with an isocyanate of the formula ZNCO, wherein Z has the meaning stated in claim 1.

6. A pharmaceutical composition comprising a compound as claimed in claim 1, together with a pharmaceutically-acceptable diluent or carrier.

**Claims for the Contracting State: AT**

1. A process for the manufacture of a compound of the formula (III),

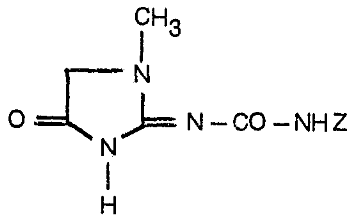

III

wherein Z stands for a thienyl radical or a thienyl radical substituted with a halogen atom, or a pharmaceutically-acceptable acid-addition salt thereof, characterised in that a compound of the formula (V):

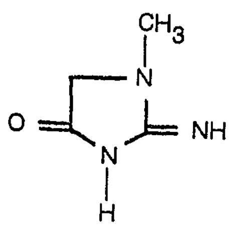

V

is reacted with an isocyanate of the formula ZNCO, wherein Z has the meaning stated above.

2. A process as claimed in claim 1, characterised in that the compound of the formula (V) is reacted with an equivalent amount or a slight excess of the isocyanate in an anhydrous aprotic organic solvent at 25 to 100°C for 1 to 10 hours.

3. A process as claimed in claim 1 or 2, characterised in that the product is a compound of the formula (III) wherein Z stands for a thienyl radical which is substituted with a chlorine atom.

4. A process as claimed in any one of claims 1 to 3, characterised in that the product is 1-(2-chloro-4-thienyl)-3-(tetrahydro-1-methyl-4-oxo-1H-imidazol-2-ylidene)urea.

**Revendications pour les Etats contractants: BE CH DE FR GB IT LU NL SE**

1. Composé de formule (III):

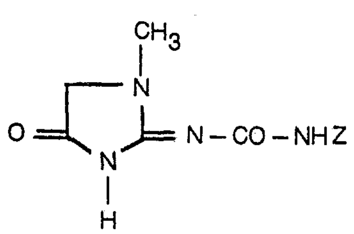

III

caractérisé en ce que Z représente un radical thiényle ou un radical thiényle substitué avec un atome d'halogène, ou un sel d'addition d'acide pharmaceutiquement acceptable de ce composé.

2. Composé suivant la revendication 1, caractérisé en ce que Z représente un radical thiényle qui est substitué avec un atome de chlore.

3. Composé suivant la revendication 1, caractérisé en ce qu'il est la 1-(2-chloro-4-thiényl)-3-(tétrahydro-1-méthyl-4-oxo-1H-imidazole-2-ylidène)urée.

4. Sel suivant la revendication 1, caractérisé en ce qu'il est un chlorhydrate, bromhydrate, phosphate, sulfate, citrate, acétate ou maléate.

5. Procédé de production d'un composé suivant la revendication 1, caractérisé en ce qu'on fait réagir un composé de formule (V):

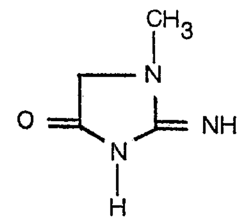

V

avec un isocyanate de formule ZNCO, dans laquelle Z a la définition donnée dans la revendication 1.

6. Composition pharmaceutique, comprenant un composé suivant la revendication 1, en association avec un diluant ou support pharmaceutiquement acceptable.

**Revendications pour l'Etat contractant: AT**

1. Procédé de production d'un composé de formule (III):

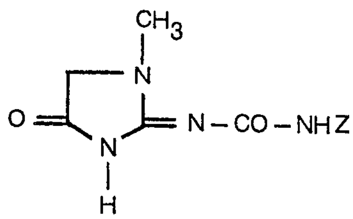

III

dans laquelle Z représente un radical thiényle ou un radical thiényle substitué avec un atome d'halogène, ou un sel d'addition d'acide pharmaceutiquement acceptable de ce composé, caractérisé en ce qu'un composé de formule (V):

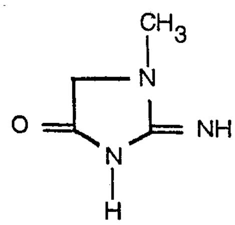

V

est amené à réagir avec un isocyanate de formule ZNCO, dans laquelle Z a la définition donnée ci-dessus.

2. Procédé suivant la revendication 1, caractérisé en ce que le composé de formule (V) est amené à réagir avec une quantité équivalente ou un léger excès de l'isocyanate dans un solvant organique aprotique anhydre à 25—100°C pendant 1 à 10 heures.

3. Procédé suivant la revendication 1 ou 2, caractérisé en ce que le produit est un composé de formule (III) dans laquelle Z représente un radical thiényle qui est substitué avec un atome de chlore. 4. Procédé suivant l'une quelconque des revendications 1 à 3, caractérisé en ce que le produit est la 1-(2-chloro-4-thiényl-3-(tétrahydro-1-méthyl-4-oxo-1H-imidazole-2-ylidène)urée.

**Patentansprüche für die Vertragsstaaten BE CH DE FR GB IT LU NL SE**

1. Verbindung der Formel (III)

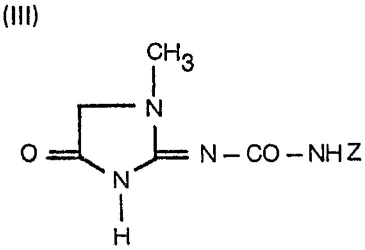

III

oder ein pharmazeutisch zulässiges Säureadditionssalz davon, dadurch gekennzeichnet, daß Z für ein Thienyl-Radikal oder ein mit einem Halogenatom substituiertes Thienyl-Radikal steht.

2. Verbindung nach Anspruch 1, dadurch gekennzeichnet, daß Z für ein Thienyl-Radikal, das mit einem Chloratom substituiert ist, steht.

3. Verbindung nach Anspruch 1, dadurch gekennzeichnet, daß es sich um 1-(2-Chloro-4-thienyl)-3-(tetrahydro-1-methyl-4-oxo-1H-imidazol-2-yliden)harnstoff handelt.

4. Salz nach Anspruch 1, dadurch gekennzeichnet, daß es ein Hydrochlorid, Hydrobromid, Phosphat, Sulfat, Citrat, Acetat oder Maleat ist.

5. Verfahren zur Herstellung einer Verbindung nach Anspruch 1, dadurch gekennzeichnet, daß eine Verbindung der Formel (V)

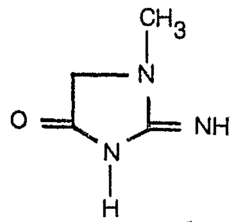

V

mit einem Isocyanat der Formel ZNCO, worin Z die in Anspruch 1 angegebene Bedeutung besitzt, umgesetzt wird.

6. Pharmazeutische Zusammensetzung, welche eine Verbindung nach Anspruch 1 gemeinsam mit einem pharmazeutisch zulässigen Verdünnungs- oder Trägermittel enthält.

**Patentansprüche für den Vertragsstaat: AT**

1. Verfahren zur Herstellung einer Verbindung der Formel (III)

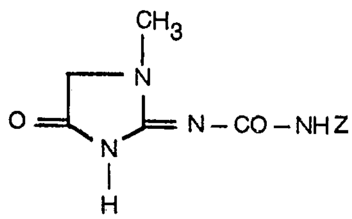

III

worin Z für ein Thienyl-Radikal oder ein durch ein Halogenatom substituiertes Thienyl-Radikal steht, oder eines pharmazeutisch zulässigen Säureadditionssalzes davon, dadurch gekennzeichnet, daß eine Verbindung der Formel (V)

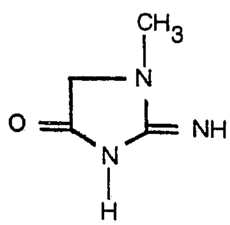

V

mit einem Isocyanat der Formel ZNCO, worin Z die oben angegebene Bedeutung besitzt, umgesetzt wird.

2. Verfahren nach Anspruch 1, dadurch gekennzeichnet, daß die Verbindung der Formel (V) mit einer äquivalenten Menge oder einem leichten Überschuß des Isocyanats in einem wasserfreien aprotischen organischen Lösungsmittel bei 25 bis 100°C während 1 bis 10 h umgesetzt wird.

3. Verfahren nach Anspruch 1 oder 2, dadurch gekennzeichnet, daß das Produkt eine Verbindung der Formel (III) ist, worin Z für ein Thienyl-Radikal das durch ein Chloratom substituiert ist, steht.

4. Verfahren nach einem der Ansprüche 1 bis 3, dadurch gekennzeichnet, daß das Produkt 1-(2-Chloro-4-thienyl)-3-(tetrahydro-1-methyl-4-oxo-1H-imidazol-2-yliden)harnstoff ist.